# EUROPEAN PATENT SPECIFICATION

(11) **EP 1 593 371 B1**
(45) Date of publication and mention of the grant of the patent: **24.08.2011**
(21) Application number: 04076359.1
(22) Date of filing: 05.05.2004
(51) Int. Cl.: A61K 8/34, A61K 8/73, A61K 8/39, A61Q 19/10, A61Q 5/02

(54) **Acid buffered skin or hair care composition**
Säuregepufferte Körperpflege Zusammensetzungen
Compositions de soin corporel contenant des tampons acides

(43) Date of publication of application: 09.11.2005
(73) Proprietor: Unilever N.V., 3013 AL Rotterdam (NL)
(72) Inventor: Piessens, Josephus Petrus, 2275 BN Voorburg (NL); Lambers, Johannes Wilhelmus Jacobus, 2564 LB Den Haag (NL)
(74) Representative: Winckels, Johannes Hubertus F.

(56) References cited:
- EP-A- 0 739 621
- EP-A- 1 136 065
- EP-A- 1 172 083
- EP-A- 1 203 574
- EP-A- 1 316 306
- EP-A2- 0 584 692
- WO-A1-96/11572
- DE-A1- 4 225 985
- JP-A- 11 310 506
- JP-A- 11 322 591
- US-A- 4 954 487
- US-A1- 2002 098 210
- US-A1- 2002 098 220
- US-A1- 2003 012 801
- US-A1- 2004 042 990

## Description

The invention relates to a composition and the use of such composition, directly or in diluted form, to the scalp, the skin and/or its appendages in order to improve the condition of the scalp, the skin and/or the skin appendages.

Skin health is of paramount importance for the body. One of the basic functions of the skin is the barrier function. Another is the regulation of heat transfer and yet another is the maintenance of the right balance in the skin micro-flora. The skin protects the body from invading micro-organisms, from out-flux of precious components like water, NMF (Natural Moisturizing Factor) and electrolytes and from in-flux of noxious substances *(e.g.* heavy metals, allergens, toxins *etc.).*

It is known in the art that products with an alkaline pH (such as traditional soaps) are detrimental to the skin condition. In particular such products cause dryness, irritation scaling and eventually fissures. To overcome this disadvantage, non-alkaline body care products have been developed. Accordingly, it has become commonly accepted that the pH of a body care product, in particular a skin or hair care product, should have a pH (when applied) of about 5.5 to 6.0 to avoid aforementioned detrimental effects to the skin surface.

EP-584692 discloses aqueous and/or alcoholic compositions containing surfactants for cleaning and conditioning hair, skin, textiles and hard surfaces containing within pH ranges of 4 - 7 aliphatic C10-C15 diols and citric acid. The compositions are useful e.g. as shampoos and shower gels.

However, there is still need for alternative body care products that do not, or at least to a lesser extent, adversely affect the skin, in particular the barrier function of the skin.

In this regard, it is important to note that the skin has its own microflora that acts as protection against external influences. Healthy skin excludes colonization of transient, potentially pathogenic micro-organisms. Normal, healthy skin flora (also known as resident or intrinsic flora) are believed to include the following principle groups of bacteria: *Staphylococci,* *Micrococci* (NB *Staphylococci* are often classified as *Micrococci),* aerobic *Coryneforms* (usually lipophilic diphteroids; NB *Brevibacteria* are often classified as *Coryneforms),* and anaerobic *Coryneforms* (e.g. *Propionibacteria).* In addition to these groups of bacteria, the yeast *Malassezia spec.* (formerly called *Pitosporumi)* can normally be found on the skin. *Demodex folliculorum,* a lipophilic mite, is also considered to be a member of the resident skin flora.

Various exogenous bacteria and/or yeasts (transient or extrinsic flora) are potentially pathogenic and may colonize skin. This occurs typically under unhealthy conditions, wherein the skin is compromised. The most notorious examples of such exogenous micro-organisms are the Gram positive, coagulase positive *Staphylococcus aureus,* the yeast *Candida albicans,* and Gram negative bacteria such as *Streptococci, Escherichia,* and *Pseudomonas specs.* These micro-organisms may live on human skin only temporarily, which is why they are frequently referred to by the terms opportunistic resident or temporary resident.

Particularly *Staphylococcus aureus,* and to a lesser extent also *Staphylococcus haemolyticus,* are harmful pathogenic micro-organisms. The increasing use of antibiotics in hospitals has resulted in significant increase in occurrence of antibiotic resistant bacteria. Methicillin Resistant *Staphylococcus Aureus* (MRSA) is such a resistant strain which presents a considerable risk to human health. Traditionally, MRSA stood for methicillin resistance but the term nowadays also refers to a multi-drug resistant group having resistance to many antibiotics traditionally used against *Staphylococcus aureus.*

On normal, healthy skin, *Staphylococcus aureus* is mostly absent and is usually recovered in about 10% of all skin isolates, especially in nasolabial areas. This is very different from unhealthy, compromised skin, where apparently conditions are such that *Staphylococcus aureus* may colonize the skin. *Staphylococcus aureus* is capable of causing various infections of the skin and other organs. These infections are common in people with frequent skin injury, particularly if the skin is dry. Representative infections are ipetico and boils (furuncles). *Staphylococcus aureus* produces entero- and exotoxins which are of proteinaceous origin and are responsible for a considerable number of diseases in men.

Of course under conditions where infections are manifest, medical treatment is urgent and antibiotic regimes have to be managed carefully. For the cosmetics industry, it is of interest to develop products which will either help to prevent the settling or regulate and minimize the occurrence of *Staphylococcus aureus* on the skin.

The development of such products, however, is associated with considerable problems as antimicrobial agents commonly used in cosmetics are not sufficiently selective. As outlined above, human skin comprises an elaborate and vitally important microflora system which includes various micro-organisms. The balance of these healthy micro-organisms should not be negatively affected by the presence of any antimicrobial agents in cosmetics, particularly not in view of the frequency with which cosmetic product are typically used by consumers. In this respect it is worthwhile noting that *Staphylococci* are the predominant group of aerobic micro-organisms that can be found on dry skin. *Staphylococcus epidermis* is part of the endogenous microflora that is considered non-pathogenic under normal conditions of health. *Staphylococcus aureus* and *Staphylococcus haemolyticus*, as discussed above, are considered to be associated with several skin ailments. Hence, selectivity is of great importance.

The present invention provides a skin or hair care product that assists in restoring the normal, healthy balance in microflora of the skin or scalp. It selectively impairs the growth of exogenous micro-organisms, particularly of pathogenic bacteria such as *Staphylococcus aureus* and *Staphylococcus haemolyticus,* or even eradicates populations of such micro-organisms completely, while not, or at least not to a (substantial) detrimental degree, affecting the intrinsic flora of the skin, most notably *Staphylococcus* *epidermis* which is closely related to the aforementioned pathogenic bacteria strains.

Accordingly, it is an object of the present invention to provide a novel cosmetic composition, in particular a topical body or skin or hair care composition (both leave-on and rinse-off), which can serve as an alternative to known cosmetic compositions and, in particular with respect to restoring the natural balance of micro-organisms present in or on human skin or scalp, to provide an improvement over known cosmetic compositions.

A cosmetic composition according to the invention is a skin or hair care composition having a pH in the range of more than.4.5 and less than 5.0, comprising an acid having a pKa in the range 3.5-5.8 in an amount of 0.5-25 wt% and an alkyl polyol in an amount of 0.01-10 wt.%, the alkyl polyol having the structure CH₈-(CH₂)ₙCH(OH)-CH₂(OH), wherein n = 3-7, or CH₃-(CH₂)ₙ-CO-(O-CH₂-CHOH-CHOH)ₘH, wherein n = 4-8 and m = 1-6.

It will be understood that combinations of one or more different alkyl polyols can also be employed.

In accordance with the invention, the alkyl polyol has the structure CH₃-(CH₂)ₙCH(OH)-CH₂(OH), wherein n = 3-7, or CH₃-(CH₂)ₙ-CO-(O-CH₂-CHOH-CHOH)ₘH, wherein n = 4-8 and m = 1-6. Highly preferred alkyl polyols are caproyl glyceride, caproyl polyglyceride, capryl glyceride, capryl polyglyceride, glyceryl caprate, caprylyl glycol, and combinations thereof. Particularly preferred are glyceryl caprate, caprylyl glycol and combinations thereof.

The amount in which an alkyl polyol is present in a composition according to the invention lies between 0.01 and 10 wt.%, more preferably between 0.1 and 5 wt.%.

Another important characteristic of a skin or hair care composition according to the invention is its pH. It has been found that a composition according to the invention brings the pH of the skin to a desired value and is at the same time very mild to the skin.

A composition according to the invention has surprisingly been found to have a beneficial effect on the barrier function of the skin in one or more ways. In particular, a composition according to the invention has been found to have a strengthening effect on at least one of the following functions: (i) the physico-chemical barrier realised by the keratinocytes and the epidermal lipids forming bi-layered sheets embedding those keratinocytes typically found in the deeper layers of the Stratum Corneum, (ii) the microbiological barrier (at the surface of the Stratum Corneum), (iii) proper homeostasis in order to maintain a strong, smooth, unbroken and flexible skin condition (in particular in the superficial layers of the Stratum Corneum).

In the skin, a pH gradient exists from the skin surface to the deepest layers. The pH at the surface is almost always lower than in the deeper parts (such as Stratum Compactum and Stratum Granulosum) where pH reaches that of the internal body fluids.

Without being bound by theory, the inventors contemplated that the positive effect of a composition according to the invention on the barrier function of the skin, is partially due to the capacity to restore and/or maintain the pH of different layers of the skin at a normal (i.e. non-pathological) value.

A composition according to the invention thus has been found to have a balancing effect on the skin, which helps to maintain the skin integrity.

The skin or hair care composition according to the invention has a pH in the range of more than 4.5 and less than 5.0, comprises at least one stabilizing acid having a pKa in the range of 3.5 to 5.8 in an amount of 0.5 to 25 wt.% to buffer the composition. More in particular, the present invention relates to a body care composition, such as a skin or hair care composition or a hair care composition, having a pH of more than 4.5 and less than 5.0 with a buffering capacity (in the range of pH 4-5) of at least 0.05 equivalent per kg, preferably 0.5 equivalent per kg, more preferably 1-10 equivalent per kg, per pH (in the same range) as it is applied. Such a product preferably comprises (I) a stabilizing acid with a pKa between 3.5 and 5.8 in an effective amount to buffer the composition and (II) at least one physiologically acceptable inorganic cation.

The pH as used herein is defined as the value as measured with a calibrated pH electrode according to ISO 4319 (1977) using a 10% dilution in demineralised water at 25 °C after 5 minutes stirring. The buffer capacity as used herein is defined as the equivalent amount of 0.1 N NaOH needed to bring the pH of an aqueous solution of the composition from pH 4 to pH 5, calculated as mol equivalents per kg product per pH. (under conditions similar to those mentioned under pH measurement).

The term stabilizing acid is used herein to describe a component that when used in a composition effectively takes up or releases H⁺ (e.g. as H₃O⁺) while maintaining approximately the pH of the composition.

A suitable amount of stabilizing acid can readily be determined by the skilled person based upon the buffering capacity of the skin, the dilution of the composition, the pH of the composition as used, the amount and the buffering capacity of the stabilizing acid.

The pH of the composition (as used) is between more than 4.5 and less than 5.0. Very good results have been achieved with a composition having a pH between 4.6 and 4.9.

The buffering capacity of the stabilizing acid(s) is preferably at least 0.1 mol equivalent per kg per pH (between pH 4 and 5), more preferably the buffering capacity is at least 0.2 mol equivalent per kg per pH, even more preferably at least 0.5 mol equivalent per kg per pH. Very good results have been achieved with a buffering capacity of at least 1 mol equivalent per kg per pH.

The stabilizing acid has a pKa in the range 3.5 to 5.8, more preferably in the range of 3.8-4.8. Preferably the stabilizing acid is uncharged to a considerable amount, typically for at least 10%, at the pH of application of the composition to the skin. It has been found that such a stabilizing acid in particular has a beneficial effect on homeostasis and/or the physico-chemical barrier. Without being bound to theory, it is thought that this effect is due to the relatively high tendency of such uncharged stabilising acid to migrate to a deeper (but still rather superficial) skin layer and restore or maintain a normal (healthy) pH value, characteristic for that layer, while creating the desired pH gradient in the skin or scalp.

Preferably, the stabilizing acid has a log octanol/water partition coefficient between -2 and +3, more preferably between -1.5 and +2.5. The log octanol/water partition coefficient is the value as obtainable by the method to establish the octanol/water partition coefficient, found as method 8 in annex V of the European Dangerous Substances Act; it basically measures the quantity of the undissociated substance in water and in n-octanol that have been fully equilibrated.

The stabilizing acid may comprise a single acidic group, two acidic groups or more than two acidic groups. Good results have *inter alia* been achieved with a composition comprising of at least one stabilizing acid selected from the group consisting of acetic, adipic, ascorbic, benzoic, buteric, enolpyruvic, glutaric, glycolic, ketovaleric, lactic, β lactic, methylbenzoic, pentanoic, phenylacetic, propionic, pyrrolcarboxylic, succinic and vinylacetic acid.

The amount of stabilizing acid is between 0.5 and 25 wt. % based upon the total weight.

To increase buffer capacity and to fix the pH, one or more other acids may be employed, which may optionally serve as stabilizing acids. Suitable examples thereof are amongst others phosphoric, hydrochloric, sulphuric, sulphonic, amino, citric, gluconic, phytic and carboxylic acids. Besides, buffers such as betain derivates and the like, can be employed.

A composition according to the invention preferably comprises one or more bi-valent and/or tri-valent inorganic cations. These cations may be present in a free ionic form or in a complex. Particular preferred bi-valent cations are calcium, magnesium, strontium, copper and zinc. A particular preferred tri-valent cation is aluminium. Calcium, magnesium, copper and zinc may enhance the physico-chemical barrier, the microbiological barrier and the homeostasis. It is assumed that any of these ions enhance the activity of one or more enzymes present in the skin, e.g. selected from the group consisting of proteases, ceramidases, glucocerebrodidases and phospholipases. It is also possible that the presence of any of these ions helps to restore mineral levels in the skin, in case of mineral-depleted skin. With respect to enhancement of the physico-chemical barrier, the inventors contemplate that calcium, magnesium, strontium and/or zinc contribute to the strength of the lipid crystal layer in the skin, in particular the deeper layers of the skin. In particular of zinc and copper it has been found that it contributes to inactivation of bacteria or even has a bactericidal effect, possibly due to inactivation of the cell-wall proteins. Further zinc and/or aluminium may serve as a pH buffer due to their capacity to form a complex with hydroxide.

The bi-valent cations are preferably present in an effective amount to enhance the enzyme activity of an enzyme involved in the keratinocyte proliferation, keratinocyte differentiation, desquamation and/or lipid breakdown.

The inorganic bi-valent and tri-valent cations are preferably present in a total concentration of 0.01 to 5 wt.%. In a leave-on product, a total concentration of 0.02 to 1.0 wt. % is highly preferred. In a rinse-off composition, a total concentration of 0.05 to 2.5 wt. % is highly preferred.

Preferably, at least one cation selected of zinc and aluminium is present in a concentration in a range, mentioned in the previous paragraph.

Particular good results have been achieved with a composition, wherein at least part of the bi-valent and/or tri-valent cations are present in combination with an organic acid that is a stabilizing acid. Especially, the lactic acid/lactate has a desirable moisturizing effect on the skin. The use of salts of the di-and tri-valent cations and anionic surfactants (normal ingredients of skin or hair care formulations) is seen as an elegant and simple way of making a preferred composition. Particularly the zinc salt of alkyl ether sulphuric acid is the preferred way of adding zinc cations. Other inorganic water soluble salts of e.g. zinc, copper or aluminium may also be used.

Preferably the cation and the organic acid-anion form a soluble complex. This has the advantage of improved availability and better penetration of the cation in the Stratum Corneum. In particular zinc and aluminium cations may form a less soluble metal hydroxide complex, if the pH is increased (in the deeper layers of the skin or when in contact with slightly alkaline water). Such a complex could serve as depot for the (metal) cation and establish a permanent low effective level. It is feasible that such precipitates serve as sun filters or block sweat gland orifices.

The inventors have found that especially a rinse-off composition combining zinc and lactic acid has markedly stronger antibacterial properties as zinc or lactic acid alone. This synergistic effect has been found during in vitro as well as in vivo experiments.

Besides the above mentioned components, other bacteriostatic compounds may be present in a composition according to the invention, e.g. to increase selectivity. Examples of such bacteriostatic compounds are chlorinated and bromated aromatic compounds, such as triclosan and bromochlorophene, quaternary compounds, such as cetylpyridinium bromide and chlorhexidine, hypothiocyanate and hypoiodide forming systems, bacteriostatic proteins, such as nisin, immunoglobulins, lactoferrin, lysozyme, and the like.

In addition, a composition according to the invention may comprise any ingredients conventionally used in skin or hair care compositions.

The composition can be used as a pharmaceutical or as a cosmetic application, for example as a rinse-off composition or as a leave-on composition.

Rinse-off compositions are medicinal or cosmetic preparations usually in the form of creams, lotions, pastes, tablets to be diluted, balms, wet tissues, powders to be diluted, gels, sticks, aerosols etc., meant to be removed shortly after application. Usually they are skin, hair, uro-genital cleaning or deodorizing preparations like (synthetic)soaps, hand, face, baby bottom's cleaners, shampoos, shower products, bath additives, make-up removers, cleaning lotions, wet travelling tissues, shaving gels, *etc.*

Leave-on compositions are medicinal or cosmetic preparations usually in the form of creams, lotions, pastes, tablets to be diluted, balms, wet tissues, powders to be diluted, gels, sticks, aerosols etc., not meant to be removed immediately; in the form of creams, lotions, pastes, balms, wet tissues, powders to be diluted, aerosols They are used for all kinds of reasons e.g. as (facial) decoration (like eye/face make up, lip stick, vanishing creams, beauty masks) or for skin care (day or night cream, talcum powder, hand cream, nipple cream, sunscreen products, baby bottom product etc) as deodorants, as aftershave, for hair care, uro-genital comfort, for footcare, for treating or preventing pimples etc.

The variety of possible medicinal and cosmetic body care compositions in which the invention can be applied, precludes the use of an all encompassing general formulation. Formulatory examples are given below. These examples are not meant to limit the scope of the invention.

### Example I

In the laboratory a number of compositions were prepared.

All preparations were done by an experienced lab technician of a development laboratory using laboratory equipment (stirrers, pH meter, homogenizer, heaters scales, incubators and refrigerator).

Batches of 1 kg of each of the six formulations (see Table 1) were prepared at ambient temperature, by dissolving the salts and the acids in 550 gram demineralised water, adjusting the pH to about 3.5 with sodium hydroxide. Next, the surfactants, the polymer, the preservative (benzoate)and glycerol were dissolved in this solution, followed by the caprylyl glycol or glyceryl caprate. Perfume was mixed with the PEG-200 hydrogenated glyceryl palmate and this mix was dissolved in the surfactant/acid aqueous solution. This solution was pH adjusted with sodium hydroxide and water was added to make-up 1 kg.

Samples were taken for stability experiments, for measurements of biophysical and microbiological effects and for consumer evaluation.

**[0]Table 1:**

| Shower gel ingredients | | | | | | |
|---|---|---|---|---|---|---|
| Chemical or INCI name | Amount in formula in % w/w | | | | | |
| | SG1* | SG2 | SG3 | SG4* | SG5 | SG6* |
| Lactic acid | 1.0 | 1.5 | 2.5 | 5 | - | - |
| Succinic acid | - | - | - | - | 4 | - |
| Glycolic acid | - | - | - | - | - | 4 |
| Calcium lactate | 0.25 | 0.1 | - | - | 0.2 | - |
| Magnesium lactate | 0.25 | 0.1 | 0.1 | 0.1 | - | - |
| Strontium chloride | - | - | 0.1 | - | - | - |
| Zinc LES | - | - | - | 0.25 | 0.2 | 0.25 |
| Aluminium chloride | - | 0,1 | - | - | - | - |
| Glyceryl caprate | 0.1 | 1.0 | | - | - | 0.5 |
| Caprylyl glycol | - | - | 0.5 | 0.1 | 1.0 | - |
| Sodium Laureth sulphate | 10 | 10 | 10 | 10 | 10 | 10 |
| Cocamido propyl betaine | 6 | 6 | 6 | 6 | 6 | 6 |
| Coco glucoside | 3 | 3 | 3 | 3 | 3 | 3 |
| PEG-200 hydrogenated glyceryl palmate | 1 | 1 | 1 | 1 | 1 | 1 |
| Styrene/acrylates copolymer | 1.5 | 1.5 | 1.5 | 1.5 | 1.5 | 1.5 |
| Glycerol | 4 | 4 | 4 | 4 | 4 | 4 |
| Sodium benzoate | 0.5 | 0.5 | 0.5 | 0.5 | 0.5 | 0.5 |
| Sodium hydroxide | qs | qs | qs | qs | qs | qs |
| Parfume | 0.4 | 0.4 | 0.4 | 0.4 | 0.4 | 0.4 |
| Aqua | ad 100 | ad 100 | ad 100 | ad 100 | ad 100 | ad 100 |
| pH of formula 5% | 4.5 | 4.7 | 4.6 | 4.4 | 4.8 | 4.3 |

| | | | | | | |
|---|---|---|---|---|---|---|
| * reference examples | | | | | | |

### Results obtained with SG4

Shower gel formula 4 (SG4) was tested for its buffer capacity and its effect on skin pH.

After application of the SG4 (during 2 minutes and rinsing-off during 20 seconds) on the underarm skin of a larger test panel, the pH of the test person's skin changed significantly during several hours. After multiple applications, the number of certain skin micro-organisms was reduced and the water barrier capacity of the skin was increased when compared with soap or water treatments.

Shower gel formula SG4 was successfully tried in consumer panels.

### Example II

### Material and methods

Via swabbing and plating-out the number of *S. aureus, S. epidermidis* and other aerobic micro flora components inhabiting the hands of 30 volunteers was established (selective plates and biochemical reactions were used for identification). The hands of these healthy volunteers had to harbor *S. aureus* before the subjects were enrolled in the test, that was set-up and executed according a protocol in accordance with good clinical practice.

On day 1 the micro flora on the hands was enumerated ("day 1 before" in the figures), the volunteers washed with the test-product and 30 minutes after drying a second sample was taken ("day 1 after" in the figures). The volunteers were instructed to use only the test-product when washing their hands during one week. On day 7 they returned to have their hand flora enumerated again ("day 7 before" and "day 7 after" in the figures). The test was performed three times with three different products.

The products tested were:
Product A (a standard synthetic bath & shower gel formulation) code: Formula SX-1315-0043.
Product B (the test product containing lactic acid (pH=4.6), a Zn salt and caprylylglycol in a bath & shower gel formulation) code: SX-1315-0055.
Product C (a soap based liquid bath & shower product) code: Tender Care Mild Soap.

The formulations of these three products are shown in table 2.

The data collected were processed via the SPSS statistical program.

**Table 2: Formulations of products A, B, and C**

| Formula: | Scientific blank SX 1315-0043 | Experimental formula SX-1315-0055 | Formula: | Commercial blank Tender Care liquid soap |
|---|---|---|---|---|
| Ingredients | pH=7.0 | pH=4.6 | Ingredients | pH=9.6 |
| Water | 77.8 | 74.9 | Water | 49.7 |
| SLES 2EO (70%) | 10.8 | 10.8 | Soap | 25.4 |
| CAPB(30%) | 5.4 | 5.4 | Propylene Glycol | 2.5 |
| APG (50%) | 3.2 | 3.2 | Glycerin | 8.0 |
| Acrylate polymer | - | 1.2 | Carbomer | 0.5 |
| Polyquaternium 7 | - | 1 | Polyquaternium 7 | 1.5 |
| Lactic acid (90%) | 0.16 | 1.1 | Skimmed Milk powder | 0.1 |
| Na Benzoate | - | 0.55 | Methyl paraben | 0.2 |
| Na Chloride | 1.13 | 0.47 | BHT | 0.05 |
| Caprylyglycol | - | 0.1 | Glycol distearate | 4.0 |
| Zinc sulphate 7 aq | - | 0.1 | KOH | 6.6 |
| NaOH (50%) | - | 0.32 | EDTA | 0.1 |
| 200PEG hydrogenated glycerylpalmitate | 1.0 | 0.5 | Vit E acetate | 0.05 |
| | | | Leucocyte extract | 0.1 |
| Perfume | 0.335 | 0.335 | Perfume | 1.1 |
| Kathon CG | 0.06 | - | d-Panthenol | 0.1 |
| Glycerine | 0.1 | - | | |

### Results

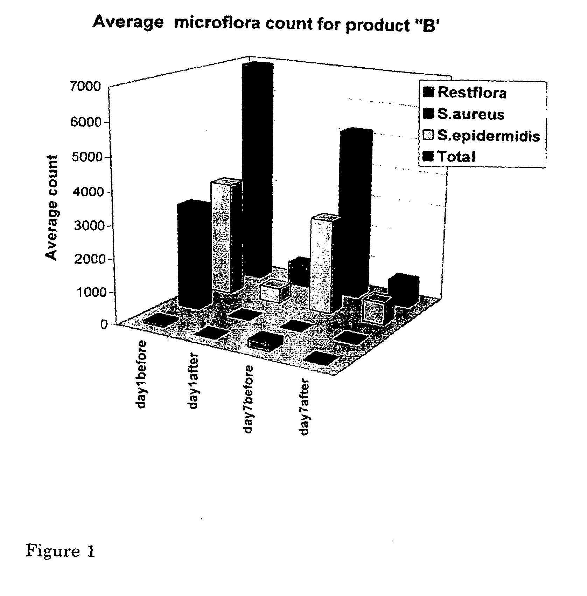

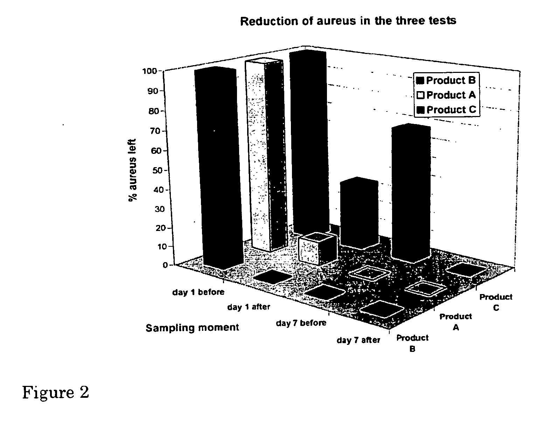

Handwashing reduces average bacterial counts in the three tests; in test B (using product SX-1315-0055) the average count of *S. aureus* is reduced dramatically after one washing. After one week's use *aureus* completely disappears, as can be seen in graph. 1. The average count of *epidermidis* is reduced after one wash, but during the subsequent week's use it recovers, so that there is no significant difference with the start value (day 1 before). The graphical representation (fig.2.) shows the relative reductions of S. aureus (the aureus start values are normalized to 100% caused by the three formulae in the three tests) in the three tests. It is clear that the reduction in test B (caused by product SX-1315-0055) is larger than that in the other tests. The difference between test B and test C is significant.

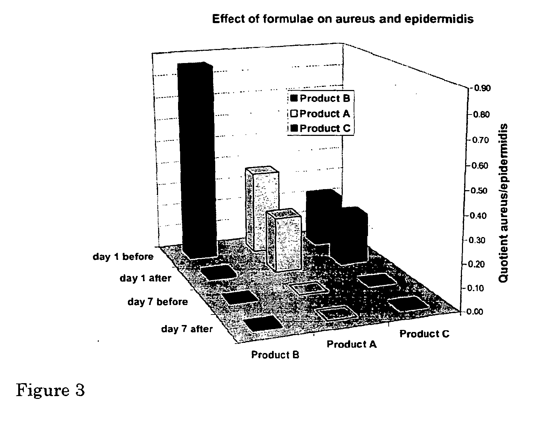

Since *S. aureus* is considered a pathogen and *S. epidermidis* is the most important representative of the resident aerobic flora, the quotient of the average count of both (the Q-value) is of crucial importance. The graph (fig.3) shows this Q-value (aureus count/epidermidis count). The reductions of the Q-value are a measure of the efficacy of the product to reduce *aureus* and at the same time maintain (or even stimulate) *epidermidis.*

Statistical analysis (ANOVA) of these reductions of the Q-values shows that in test B (product SX-1315-0055) the reductions are larger than in test A (product SX-1315-0043) and than in test C (product Tender Care Mild Soap).

### Conclusions

Use of product B on the hands of "healthy" people carrying *S. aureus* (considered to be a pathogen) results in a near eradication of the organism after one application. Further application leads to complete disappearance of *S. aureus* whilst it has no negative influence on *S. epidermidis,* (main part of the indigenous skin flora).

Compared to reference formulations, product B causes greater reduction of *S. aureus* and better maintenance of *S. epidermidis.*

## Claims

1. Skin or hair care composition having a pH in the range of more than.4.5 and less than 5.0, comprising an acid having a pKa in the range 3.5-5.8 in an amount of 0.5-25 wt% and an alkyl polyol in an amount of 0.01-10 wt.%, the alkyl polyol having the structure CH₈-(CH₂)ₙCH(OH)-CH₂(OH), wherein n = 3-7, or CH₈-(CH₂)ₙ-CO-(O-CH₂-CHOH-CHOH)ₘH, wherein n = 4-8 and m = 1-6.

2. Composition according to claim 2, wherein the alkyl polyol is chosen from the group of caproyl glyceride, caproyl polyglyceride, capryl glyceride, capryl polyglyceride, glyceryl caprate, caprylyl glycol, and combinations thereof.

3. Composition according claim 1 or 2, wherein the alkyl polyol is present in an amount of from 0.1 to 5 wt.%.

4. Composition according to any one of the preceding claims, wherein the pKa of the acid is.3.8-4.8.

5. Composition according to any one of the preceding claims, wherein the buffering capacity of the acid, defined as the equivalent amount of 0.1 N NaOH needed to bring the pH of an aqueous solution of the composition from pH 4 to pH 5, is at least 0.05 mol equivalent 0.1 N NaOH per kg between pH 4 and 5, preferably at least 0.5 mol equivalent 0.1 N NaOH per kg between pH 4 and 5, more preferably 1-10 mol equivalent 0.1 N NaOH per kg between pH 4 and 5, the pH measured with a calibrated pH electrode according to ISO 4319 (1977) using a 10% dilution in demineralised water at 25 °C after 5 minutes stirring.

6. Composition according to any one of the preceding claims, wherein the acid is for at least 10% uncharged at the pH of the composition at application.

7. Composition according to any one of the preceding claims, wherein the acid has a log octanol/water partition coefficient (Po/w) between -1.5 and +2, preferably in the range of -1 to +1.5.

8. Composition according to any one of the preceding claims, comprising at least one acid selected from the group consisting of acetic, adipic, ascorbic, benzoic, butyric, enol pyruvic, glutaric, glycolic, ketovaleric, lactic, 8 lactic,
methylbenzoic, pentanoic, phenylacetic, propionic, pyrrole carboxylic, succinic and vinylacetic acid.

9. Composition according to claim 8, wherein the acid is selected from the group consisting of lactic acid, succinic acid and glutaric acid.

10. Composition according to any one of the preceding claims, wherein the amount of the acid is 1-15 wt. %.

11. Composition according to any one of the preceding claims, further comprising at least one physiologically acceptable inorganic cation.

12. Composition according to claim 11, wherein the cation is selected from the group consisting of bivalent cations and trivalent cations.

13. Composition according to claim 12, comprising aluminium as a trivalent cation.

14. Composition according to any one of the claims 11-13, comprising at least one bivalent cation selected from the group of calcium, magnesium, strontium, copper and zinc.

15. Composition according to claim 14, wherein the bivalent cation is zinc.

16. Composition according to any one of the claims 12-15, wherein the total concentration of bivalent and/or trivalent cation(s) is 0.05 to 5 wt.%.

17. Composition according to any one of the claims 12-16 where the bi- and/or trivalent cation is added in the form of a salt of a acid or as a salt of an anionic surfactant appropriate to the composition.

18. Composition according to any one of the claims 11-17, wherein the bi- and/or tri-valent cation has a total concentration of 0.01 to 5 wt.%.

19. Composition according to any one of the claims 11-18, wherein the product is a rinse-off product, and the bi and/or trivalent cation has a total concentration of 0.05.2.5 wt.%.

20. Composition according to any of the preceding claims comprising an additional bacteriostatic compound.

21. Composition according to any one of the preceding claims, wherein the composition is a "rinse off' product or a "leave on" product suitable to be applied to the skin or its appendages, directly in undiluted form or after dilution.

22. A composition according to any one of the preceding claims for use in a method to reduce the presence of possible pathogenic micro-organisms relatively to the indigenous microflora of skin, scalp and/or skin appendages.

23. A composition according to claim 22, for use in a method to stimulate the natural anti-microbial defence system, physico-chemical barrier function, homeostasis, cell renewal, barrier recovery, lipid barrier function, keratinocyte proliferation, keratinocyte differentiation and/or proper lipid- and protein processing.

24. A composition according to any one of the preceding claims, for use in a method wherein the composition is applied onto human skin or scalp, for the purpose of assisting in restoring the normal, healthy balance in microflora of the skin or scalp.

## Patentansprüche

1. Haut- oder Haarpflegezusammensetzung mit einem pH-Wert im Bereich von mehr als 4,5 und weniger als 5,0, umfassend eine Säure mit einem pKa im Bereich von 3,5-5,8 in einer Menge von 0,5-25 Gew.-% und einen Alkylpolyol in einer Menge von 0,01-10 Ges.-%, wobei der Alkylpolyol die Struktur CH₃-(CH₂)ₙCH(OH)-CH₂(OH), wobei n = 3-7 ist, oder CH₃-(CH₂)ₙ-CO-(O-CH₂-CHOH-CHOH)ₘH, wobei n = 4-8 ist und m = 1-6 ist, aufweist.

2. Zusammensetzung gemäß Anspruch 1, wobei das Alkylpolyol aus der Gruppe ausgewählt ist, die aus Caproylglycerid, Caproylpolyglycerid, Caprylglycerid, Caprylpolyglycerid, Glycerylcaprat, Caprylylglycol und Kombinationen davon besteht.

3. Zusammensetzung gemäß Anspruch 1 oder 2, wobei das Alkylpolyol in einer Menge von 0,1 bis 5 Gew.-% vorhanden ist.

4. Zusammensetzung gemäß einem der vorstehenden Ansprüche, wobei der pKa der Säure 3,8-4,8 beträgt.

5. Zusammensetzung gemäß einem der vorstehenden Ansprüche, wobei die Pufferkapazität der Säure, die als äquivalente Menge von 0,1 N NaOH, die notwendig ist, um den pH-Wert einer wässrigen Lösung der Zusammensetzung von pH 4 auf pH 5 zu bringen, definiert ist, wenigstens 0,05 Moläquivalent 0,1 N NaOH pro kg zwischen pH 4 und 5, vorzugsweise wenigstens 0,5 Moläquivalent 0,1 N NaOH pro kg zwischen pH 4 und 5, besonders bevorzugt 1-10 Moläquivalent 0,1 N NaOH pro kg zwischen pH 4 und 5, beträgt, wobei der pH-Wert mit einer geeichten pH-Elektrode gemäß ISO 4319 (1977) unter Verwendung einer 10%-igen Verdünnung in demineralisiertem Wasser bei 25 °C nach 5 Minuten Rühren gemessen wird.

6. Zusammensetzung gemäß einem der vorstehenden Ansprüche, wobei die Säure bei dem pH-Wert, bei dem die Zusammensetzung angewendet wird, zu wenigstens 10% ungeladen ist.

7. Zusammensetzung gemäß einem der vorstehenden Ansprüche, wobei die Säure einen logarithmischen Octanol/Wasser-Verteilungskoeffizienten (Po/w) zwischen -1,5 und +2, vorzugsweise im Bereich von -1 bis +1,5, aufweist.

8. Zusammensetzung gemäß einem der vorstehenden Ansprüche, die wenigstens eine Säure umfasst, die aus der Gruppe ausgewählt ist, die aus Essig-, Adipin-, Ascorbin-, Benzoe-, Butter-, Enolbrenztrauben-, Glutar-, Glycol-, Ketovalerian-, Milch-, Deltamilch-, Methylbenzoe-, Pentan-, Phenylessig-, Propion-, Pyrrolcarbon-, Bernstein- und Vinylessigsäure besteht.

9. Zusammensetzung gemäß Anspruch 8, wobei die Säure aus der Gruppe ausgewählt ist, die aus Milchsäure, Bernsteinsäure und Glutarsäure besteht.

10. Zusammensetzung gemäß einem der vorstehenden Ansprüche, wobei die Menge der Säure 1-15 Gew.-% beträgt.

11. Zusammensetzung gemäß einem der vorstehenden Ansprüche, die weiterhin wenigstens ein physiologisch annehmbares anorganisches Kation umfasst.

12. Zusammensetzung gemäß Anspruch 11, wobei das Kation aus der Gruppe ausgewählt ist, die aus zweiwertigen Kationen und dreiwertigen Kationen besteht.

13. Zusammensetzung gemäß Anspruch 12, die Aluminium als dreiwertiges Kation umfasst.

14. Zusammensetzung gemäß einem der Ansprüche 11 bis 13, die wenigstens ein zweiwertiges Kation umfasst, das aus der Gruppe ausgewählt ist, die aus Calcium, Magnesium, Strontium, Kupfer und Zink besteht.

15. Zusammensetzung gemäß Anspruch 14, wobei es sich bei dem zweiwertigen Kation um Zink handelt.

16. Zusammensetzung gemäß einem der Ansprüche 12 bis 15, wobei die gesamte Konzentration von zweiwertigen und/oder dreiwertigen Kationen 0,05 bis 5 Gew.-% beträgt.

17. Zusammensetzung gemäß einem der Ansprüche 12 bis 16, wobei das zwei- und/oder dreiwertige Kation in Form eines Salzes einer Säure oder als Salz eines anionischen Tensids, das für die Zusammensetzung geeignet ist, hinzugefügt wird.

18. Zusammensetzung gemäß einem der Ansprüche 11 bis 17, wobei das zwei- und/oder dreiwertige Kation eine Gesamtkonzentration von 0,01 bis 5 Gew.-% aufweist.

19. Zusammensetzung gemäß einem der Ansprüche 11 bis 18, wobei das Produkt ein auszuspülendes/abzuwaschendes Mittel ist und das zwei-und/oder dreiwertige Kation eine Gesamtkonzentration von 0,05 bis 2,5 Gew.-% aufweist.

20. Zusammensetzung gemäß einem der vorstehenden Ansprüche, die eine zusätzliche bakteriostatische Verbindung umfasst.

21. Zusammensetzung gemäß einem der vorstehenden Ansprüche, wobei die Zusammensetzung ein "auszuspülendes/abzuwaschendes" Mittel oder ein Mittel, "das auf der Haut/in den Haaren verbleibt", ist und für die Auftragung auf die Haut oder ihre Anhängsel direkt in unverdünnter Form oder nach Verdünnung geeignet ist.

22. Zusammensetzung gemäß einem der vorstehenden Ansprüche zur Verwendung in einem Verfahren zum Reduzieren der Anwesenheit möglicher pathogener Mikroorganismen relativ zur residenten Mikroflora der Haut, Kopfhaut und/oder der Hautanhängsel.

23. Zusammensetzung gemäß Anspruch 22 zur Verwendung in einem Verfahren zum Stimulieren des natürlichen antimikrobiellen Abwehrsystems, der physikalisch-chemischen Barrierefunktion, der Homöostase, der Zellerneuerung, der Barriereregeneration, der Lipidbarrierefunktion, der Keratinocytenproliferation, der Keratinocytendifferenzierung und/oder der richtigen Lipid- und Proteinprozessierung.

24. Zusammensetzung gemäß einem der vorstehenden Ansprüche zur Verwendung in einem Verfahren, bei dem die Zusammensetzung auf die menschliche Haut oder Kopfhaut aufgetragen wird, um die Wiederherstellung des normalen gesunden Gleichgewichts in der Mikroflora der Haut oder Kopfhaut zu unterstützen.

## Revendications

1. Composition pour le soin des cheveux ou de la peau présentant un pH supérieur à 4,5 et inférieur à 5,0, comprenant un acide présentant un pKa de l'ordre de 3,5 à 5,8 dans une proportion de 0,5 à 25 % en poids et contenant un polyol alkylique en une proportion de 0,01 à 10 % en poids, le polyol alkylique présentant la structure CH₃(CH₂)ₙCH(OH)-CH₂(OH), dans laquelle n = 3 à 7, ou présentant la structure CH₃(CH₂)ₙCO-(O-CH₂-CHOH-CHOH)ₘH, dans laquelle n = 4 à 8 et m = 1 à 6.

2. Composition selon la revendication 1, dans laquelle le polyol alkylique est choisi dans le groupe comprenant les caproyl glycéride, caproyl polyglycéride, capryl glycéride, capryl polyglycéride, glycéryl caprate, caprylyl glycol, ainsi que leurs combinaisons.

3. Composition selon la revendication 1 ou 2, dans laquelle le polyol alkylique est présent en une proportion comprise entre 0,1 et 5 % en poids.

4. Composition selon l'une quelconque des revendications précédentes, dans laquelle le pKa de l'acide est de 3,8 à 4,8.

5. Composition selon l'une quelconque des revendications précédentes, dans laquelle la capacité de tampon de l'acide, définie en tant que quantité équivalente de NaOH 0,1 N nécessaire pour porter le pH d'une solution aqueuse de la composition d'un pH 4 au pH 5 est au moins de 0,5 équivalent molaire de NaOH 0,1 N par kg pour un pH compris entre 4 et 5, de préférence au moins de 0,5 équivalent molaire de NaOH 0,1 N par kg pour un pH compris entre 4 et 5, plus particulièrement de 1 à 10 équivalents molaires de NaOH 0,1 N par kg pour un pH compris entre 4 et 5, le pH étant mesuré à l'aide d'une électrode de pH calibrée selon la norme ISO 4319 (1977) utilisant une dilution de 10 % dans de l'eau déminéralisée à 25°C après 5 minutes d'agitation.

6. Composition selon l'une quelconque des revendications précédentes, dans laquelle l'acide est pour au moins 10 % non chargé au pH de la composition lors de l'application.

7. Composition selon l'une quelconque des revendications précédentes, dans laquelle l'acide présente un coefficient logarithmique de partition octanol/eau (Po/w) compris entre -1,5 et +2, de préférence compris entre -1 et +1,5.

8. Composition selon l'une quelconque des revendications précédentes, comprenant au moins un acide choisi dans le groupe comprenant les acides acétique, adipique, ascorbique, benzoïque, butyrique, énolpyruvique, glutarique, glycolique, cetovalérique, lactique, β-lactique, méthylbenzoïque, pentanoïque, phénylacétique, propionique, pyrrole carboxylique, succinique et vinylacétique.

9. Composition selon la revendication 8, dans laquelle l'acide est choisi dans le groupe comprenant l'acide lactique, l'acide succinique et l'acide glutarique.

10. Composition selon l'une quelconque des revendications précédentes, dans laquelle la quantité d'acide est comprise entre 1 et 15 % en poids.

11. Composition selon l'une quelconque des revendications précédentes, comprenant en outre au moins un cation inorganique acceptable du point de vue physiologique.

12. Composition selon la revendication 11, dans laquelle le cation est choisi dans le groupe comprenant les cations bivalents et les cations trivalents.

13. Composition selon la revendication 12, comprenant l'aluminium en tant que cation trivalent.

14. Composition selon l'une quelconque des revendications 11 à 13, comprenant au moins un cation bivalent choisi dans le groupe comprenant le calcium, le magnésium, le strontium, le cuivre et le zinc.

15. Composition selon la revendication 14, dans laquelle le cation bivalent est le zinc.

16. Composition selon l'une quelconque des revendications 12 à 15, dans laquelle la concentration totale en cation(s) bivalent et/ou trivalent est comprise entre 0,05 et 5 % en poids.

17. Composition selon l'une quelconque des revendications 12 à 16, dans laquelle le cation bi et/ou trivalent est ajouté sous la forme d'un sel d'un acide ou d'un sel d'un agent tensioactif anionique convenable à l'égard de la composition.

18. Composition selon l'une quelconque des revendications 11 à 17, dans laquelle le cation bi et/ou trivalent présente une concentration totale de 0,01 à 5% en poids.

19. Composition selon l'une quelconque des revendications 11 à 18, dans laquelle le produit est un produit d'élimination par rinçage et le cation bi et/ou trivalent présente une concentration totale de l'ordre de 0,05 à 2,5 % en poids.

20. Composition selon l'une quelconque des revendications précédentes, comprenant un composé bactériostatique supplémentaire.

21. Composition selon l'une quelconque des revendications précédentes, dans laquelle la composition est un produit d'élimination par rinçage ou un produit d'élimination par séparation susceptible d'être appliqué sur la peau ou ses appendices directement sous forme non diluée, ou après dilution.

22. Une composition selon l'une quelconque des revendications précédentes, destinée à être utilisée dans un procédé pour réduire la présence de micro-organismes pathogènes éventuels pour ce qui concerne la microflore indigène de la peau, du cuir chevelu et/ou de ses appendices.

23. Une composition selon la revendication 22, destinée à être utilisée dans un procédé pour stimuler le système de défense anti-microbien naturel, la fonction de barrière physico-chimique, l'homéostase, le renouvellement cellulaire, la récupération de barrière, la fonction de barrière lipidique, la prolifération kératinocyte, la différenciation kératinocyte et/ou le traitement protéinolipidique convenable.

24. Une composition selon l'une quelconque des revendications précédentes, destinée à être utilisée dans un procédé dans lequel la composition appliquée à la peau ou au cuir chevelu d'un homme dans le but de contribuer à restaurer l'équilibre de santé normal de la microflore de la peau ou du cuir chevelu.
